# EUROPÄISCHE PATENTSCHRIFT

(11) **EP 2 294 060 B1**
(45) Veröffentlichungstag und Bekanntmachung des Hinweises auf die Patenterteilung: **07.05.2014**
(21) Anmeldenummer: 09728771.8
(22) Anmeldetag: 23.03.2009
(51) Int. Cl.: C07D 307/60, C07C 51/573

(54) **VERFAHREN ZUR ABTRENNUNG VON FUMARSÄURE UND ANDEREN NEBENKOMPONENTEN BEI DER HERSTELLUNG VON MALEINSÄUREANHYDRID**
PROCESS FOR SEPARATING OFF FUMARIC ACID AND OTHER MINOR COMPONENTS DURING THE PRODUCTION OF MALEIC ANHYDRIDE
PROCÉDÉ POUR SÉPARER L ACIDE FUMARIQUE D AUTRES COMPOSANTS SECONDAIRES LORS DE LA PRODUCTION D ANHYDRIDE D ACIDE MALÉIQUE

(30) Priorität: 01.04.2008 EP 08153909
(43) Veröffentlichungstag der Anmeldung: 16.03.2011
(73) Patentinhaber: BASF SE, 67056 Ludwigshafen (DE)
(72) Erfinder: WINDECKER, Gunther, 67067 Ludwigshafen (DE); WEIGUNY, Jens, 67251 Freinsheim (DE); WECK, Alexander, 67251 Freinsheim (DE); DAHLHOFF, Ellen, 67117 Limburgerhof (DE); WEIßKER, Wolf-Steffen, 67245 Lambsheim (DE); HEILEK, Jörg, 69245 Bammental (DE); KRUG, Thomas, 67550 Worms (DE); FREYBERGER, Ralf, 200335 Shanghai (CN)
(86) Internationale Anmeldenummer: PCT/EP2009/053338
(87) Internationale Veröffentlichungsnummer: WO 2009/121735

(56) Entgegenhaltungen:
- WO-A-96/29323
- US-A- 4 118 403

## Beschreibung

Die vorliegende Erfindung betrifft ein Verfahren zur Verringerung von Fumarsäureablagerungen bei der Herstellung von Maleinsäureanhydrid durch heterogenkatalytische Oxidation eines Kohlenwasserstoffs ausgewählt aus der Gruppe Benzol, n-Butan, n-Buten und 1,3-Butadien, mit molekularem Sauerstoff in Gegenwart eines Vanadium, Phosphor und Sauerstoff enthaltenden Katalysators, umfassend
(a) die Absorption von Maleinsäureanhydrid aus dem Rohproduktgemisch in einem Absorptionsmittel enthaltend ein organisches Lösungsmittel in einer Absorptionskolonne;
(b) die Desorption des Maleinsäureanhydrids von dem in Schritt (a) erhaltenen, mit Maleinsäureanhydrid angereicherten Absorptionsmittel in einer Desorptionskolonne; und
(c) die vollständige oder teilweise Rückführung des in Schritt (b) von Maleinsäureanhydrid abgereicherten Absorptionsmittels zu Schritt (a).

Das erfindungsgemäße Verfahren dient zur Verbesserung der großtechnischen Herstellung von Maleinsäureanhydrid. Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Maleinsäureanhydrid kann durch Partialoxidation von Kohlenwasserstoffen, insbesondere Benzol oder C₄-Kohlenwasserstoffe, wie 1,3-Butadien, n-Butene oder n-Butan, gewonnen werden. Die Reaktion ist stark exotherm und bedarf einer ausreichenden Abfuhr der Reaktionswärme. Im Allgemeinen führt man die Umsetzung in einem Rohrbündelreaktor mit Salzkreislauf oder einem Wirbelbett durch. Das bei der Umsetzung gebildete Maleinsäureanhydrid wird üblicherweise aus dem gebildeten Rohproduktgemisch in einem Lösungsmittel absorbiert. Dabei werden neben Maleinsäureanhydrid auch weitere im Rohproduktgemisch enthaltene Komponenten absorbiert, wie zum Beispiel auch das bei der Oxidation gebildete Wasser. Das Wasser reagiert dabei zum Teil mit dem Maleinsäureanhydrid zu Maleinsäure, die wiederum zum Teil zu Fumarsäure isomerisiert. Fumarsäure ist eine in Wasser oder organischen Lösemitteln sehr schlecht lösliche Dicarbonsäure, die Ablagerungen bildet und dadurch Anlagenteile wie zum Beispiel Kolonnen, Wärmetauscher, Pumpen, Rohre und dergleichen verstopfen kann.

Um solche durch Fumarsäure verursachten Verstopfungen zu vermeiden, gibt es im Stand der Technik bereits Vorschläge.

So ist in WO 96/029,323 beschrieben, das Fumarsäure enthaltende Absoprtionsmittel nach der Herausstrippung von Maleinsäureanhydrid mit einem wässrigen Extraktionsmittel zu waschen, um so Ablagerungen zu vermeiden. Nachteilig an diesem Verfahren ist der hohe Aufwand, der notwendig ist, um das Waschwasser in eine großtechnische Anlage zur Herstellung von C₄-Dicarbonsäuren oder deren Derivaten einzumischen und die Phasen wieder zu trennen. Zudem entstehen durch den unvermeidlichen Verlust von wertvollem Produkt und Lösemittel hohe Kosten. Ferner wird durch den zusätzlichen Wassereintrag in den Prozess die Fumarsäurebildung noch verstärkt.

In DE-Az. 10 2006 024 903.8 wird vorgeschlagen, das Fumarsäure enthaltende Absoprtionsmittel nach der Herausstrippung von Maleinsäureanhydrid ganz oder teilweise katalytisch zu hydrieren und ganz oder teilweise in die Absorptionsstufe zurückzuführen. US 4 118 403 lehrt die Abtrennung von Fumarsäure aus dem von Maleinsäureanhydrid angereicherten Absorptionsmittel durch Abkühlung und Filtrierung der ausgefallenen Fumarsäure-Partikel.

Ausgehend von diesem Stand der Technik lag der vorliegenden Erfindung die Aufgabe zugrunde, Fumarsäureablagerungen auf Anlagenteilen und dadurch verursachte Verstopfungen, Ausbau- und Reinigungsarbeiten sowie Abschaltungen bei der Herstellung von Maleinsäureanhydrid mit möglichst wenig technischem Aufwand und ohne Auftreten der oben genannten Nachteile deutlich zu verringern.

Demgemäß wurde ein Verfahren zur Verringerung von Fumarsäureablagerungen bei der Herstellung von Maleinsäureanhydrid durch heterogenkatalytische Oxidation eines Kohlenwasserstoffs ausgewählt aus der Gruppe Benzol, n-Butan, n-Buten und 1,3-Butadien, mit molekularem Sauerstoff in Gegenwart eines Vanadium, Phosphor und Sauerstoff enthaltenden Katalysators, umfassend
(a) die Absorption von Maleinsäureanhydrid aus dem Rohproduktgemisch in einem Absorptionsmittel enthaltend ein organisches Lösungsmittel in einer Absorptionskolonne;
(b) die Desorption des Maleinsäureanhydrids von dem in Schritt (a) erhaltenen, mit Maleinsäureanhydrid angereicherten Absorptionsmittel in einer Desorptionskolonne; und
(c) die vollständige oder teilweise Rückführung des in Schritt (b) von Maleinsäureanhydrid abgereicherten Absorptionsmittels zu Schritt (a), gefunden, das dadurch gekennzeichnet ist, dass man
(d) die Gesamt- oder Teilmenge des in Schritt (b) von Maleinsäureanhydrid abgereicherten Absorptionsmittels zur gezielten Ausfällung der Fumarsäure soweit abkühlt und/oder durch Verdampfung eines Teils des Absorptionsmittels aufkonzentriert, dass die Differenz zwischen der Konzentration an Fumarsäure im Rückführstrom am Ausgang der Desorptionskolonne c(FA, Ausgang Desorptionskolonne) unter den dort vorliegenden Bedingungen in Gew.-ppm und der Gleichgewichtskonzentration an Fumarsäure gemäß der Löslichkeitskurve nach der Abkühlung und/oder Verdampfung eines Teils des Absorptionsmittels c(FA, Gleichgewicht nach Abkühlung/Verdampfung) in Gew.-ppm größer oder gleich 250 Gew.-ppm beträgt;
(e) die durch die Maßnahmen aus Schritt (d) als Feststoff ausgefallene Fumarsäure ganz oder teilweise kontinuierlich oder diskontinuierlich aus der Absorptionsmittelrückführung entfernt; und
(f) das an Fumarsäure abgereicherte Absorptionsmittel aus Schritt (e) ganz oder teilweise zu Schritt (a) rückführt.

Das erfindungsgemäße Verfahren verringert deutlich unerwünschte Fumarsäureablagerungen durch eine gezielte Ausfällung der Fumarsäure in der Rückführung des Absorptionsmittels durch die erfindungsgemäßen Maßnahmen und die Entfernung von ausgefallener Fumarsäure.

Erfindungsgemäß wurde überraschend gefunden, dass die Fumarsäure unter den vorliegenden Bedingungen der heterogenkatalytischen Herstellung von Maleinsäureanhydrid durch Kohlenwasserstoff-Oxidation außerordentlich stark zur Bildung von Übersättigung neigt. So fällt diese entgegen der Erwartung des Fachmanns beispielsweise beim Abkühlen des von Maleinsäureanhydrid abgereicherten Absorptionsmittels aus Schritt (b) nicht entsprechend der Löslichkeitskurve aus, sondern bildet selbst in Gegenwart von vorhandener kristalliner Fumarsäure eine hoch übersättigte Lösung. Die Übersättigung kann mehrere hundert Gew.-ppm, teilweise auch sogar über eintausend Gew.-ppm und damit ein Vielfaches der Löslichkeit betragen. Entsprechendes gilt auch für den Fall der Aufkonzentrierung durch Verdampfung eines Teils des Absorptionsmittels. Dieses überraschende Verhalten führt dazu, dass die Fumarsäure nach den Maleinsäureanhydrid-Herstellverfahren des Standes der Technik scheinbar unkontrolliert in der Absorptionsmittelrückführung und nachgelagerten Anlageteilen ausfällt und dabei mit der Zeit Apparate und Rohrleitungen verstopft.

Erfindungsgemäß ist es durch die genannten Maßnahmen gelungen, die Fumarsäure trotz ausgeprägter Neigung zur Übersättigung gezielt auszufällen. Die gezielte Ausfällung wird durch die in Schritt (d) genannten Maßnahmen der gezielten Abkühlung und/oder gezielten Aufkonzentrierung durch Verdampfung eines Teils des Absorptionsmittels erreicht, wobei durch die genannten Maßnahmen gezielt eine Differenz zwischen der Konzentration an Fumarsäure im Rückführstrom am Ausgang der Desorptionskolonne c(FA, Ausgang Desorptionskolonne) unter den dort vorliegenden Bedingungen in Gew.-ppm und der Gleichgewichtskonzentration an Fumarsäure gemäß der Löslichkeitskurve nach der Abkühlung und/oder Verdampfung eines Teils des Absorptionsmittels c(FA, Gleichgewicht nach Abkühlung/Verdampfung) in Gew.-ppm von größer oder gleich 250 Gew.-ppm eingestellt wird. Die Obergrenze der Differenz entspricht bei einer minimalen Gleichgewichtskonzentration an Fumarsäure gemäß der Löslichkeitskurve von 0 Gew.-ppm bei entsprechend niedriger Temperatur somit der Konzentration an Fumarsäure im Rückführstrom am Ausgang der Desorptionskolonne c(FA, Ausgang Desorptionskolonne). Vorteilhaft stellt man eine Differenz von c(FA, Ausgang Desorptionskolonne) minus c(FA, Gleichgewicht nach Abkühlung/Verdampfung) von größer oder gleich 350 und bevorzugt größer oder gleich 500. In besonderen Fällen, beispielsweise bei relativ hohen Konzentrationen an Fumarsäure im Absorptionsmittel von in der Regel oberhalb 1500 Gew.-ppm, ist es vorteilhaft ein Differenz von c(FA, Ausgang Desorptionskolonne) minus c(FA, Gleichgewicht nach Abkühlung/Verdampfung) von besonders bevorzugt größer oder gleich 700, ganz besonders bevorzugt größer oder gleich 1000 und insbesondere größer oder gleich 1500 Gew.-ppm einzustellen. Bevorzugt beträgt die Differenz kleiner oder gleich 5000 und besonders bevorzugt kleiner oder gleich 3000 Gew.-ppm.

Die Konzentration an Fumarsäure im Rückführstrom am Ausgang der Desorptionskolonne c(FA, Ausgang Desorptionskolonne) kann auf einfache Art und Weise analytisch bestimmt werden. Die Analyse kann beispielsweise mittels kalibrierter Gaschromatographie erfolgen. Zur Kalibrierung wird bevorzugt ein interner Standard, wie beispielsweise Diethylenglykoldimethylether, verwendet. Vor der gaschromatographischen Analyse wird die Probe im Allgemeinen homogenisiert, d.h. in einem Lösungsmittel aufgenommen. Ein hierfür bestens geeignetes Lösungsmittel ist beispielsweise N,N-Dimethylformamid. Nach der Homogenisierung wird die Probe bevorzugt mit einem geeigneten Silylierungsmittel, beispielsweise N,O-Bis-trimethylsilyl-trifluoroacetamid (BSTFA) silyliert. Als Trennsäulen eignen sich besonders gut Kapillarsäulen mit 100% Dimethylpolysiloxan (z. B. Typ DB-1 der Fa.- Agilent) oder (14%-Cyanopropyl-phenyl)-methylpolysiloxan, (z. B. Typ DB-1701 der Fa.- Agilent) mit bevorzugt jeweils 60 m Länge, 0,32 mm inneren Durchmesser und 1 µm Filmdicke.

Unter dem Ausgang der Desorptionskolonne ist der Ort zu verstehen, an dem der Strom die eigentliche Kolonne verlässt. Bevorzugt geschieht dies über den Sumpfabzug. Aus diesem sind auch die Proben für die analytische Konzentrations-Bestimmung zu entnehmen.

Die Gleichgewichtskonzentration an Fumarsäure gemäß der Löslichkeitskurve nach der Abkühlung und/oder Verdampfung eines Teils des Absorptionsmittels c(FA, Gleichgewicht nach Abkühlung/Verdampfung) lässt sich sehr einfach aus der entsprechenden Löslichkeitskurve unter Berücksichtigung der vorliegenden Temperatur entnehmen. Die Kurve der temperaturabhängigen Löslichkeit der Fumarsäure im verwendeten, gegebenenfalls teileingedampften Absorptionsmittel (Löslichkeitskurve) kann experimentell nach der folgenden Methode bestimmt werden:
(1) Das Absorptionsmittel, in dem die temperaturabhängige Löslichkeit der Fumarsäure bestimmt werden soll, wird in einem temperierbaren Rührgefäß in gerührtem Zustand auf 0°C abgekühlt.
(2) In das auf 0°C abgekühlte Absorptionsmittel wird dann etwa 1 Gew.-% Fumarsäure als Reinstoff, mindestens jedoch das Doppelte der zu erwartenden und zu messenden Maximallöslichkeit zugegeben.
(3) Nach Zugabe der Fumarsäure wird die erhaltene Mischung 24 Stunden bei 0°C gerührt.
(4) Soll die Löslichkeit bei 0°C bestimmt werden, kann nach Ablauf der 24-stündigen Rührzeit eine Probe aus der gerührten Suspension gezogen werden. Die Probenahme erfolgt über eine Spritze, die mit einem Spritzenaufsatz-Filter zum Zurückhalten der ungelösten Fumarsäure versehen ist. Als Spritzenaufsatz-Filter wird ein Membranfilter mit 0,2 Mikrometer Porenweite verwendet. Die über das Filter gezogene, feststofffreie Flüssigkeitsprobe enthält gelöste Fumarsäure. Deren Konzentration wird analog der bei der Bestimmung von c(FA, Ausgang Desorptionskolonne) beschriebenen Analysenmethode bestimmt. Der analysierte Gehalt an Fumarsäure entspricht der Löslichkeit von Fumarsäure im eingesetzten Absorptionsmittel bei 0°C.
(5) Soll die Löslichkeit bei einer Temperatur oberhalb 0°C ermittelt werden, wird die aus Punkt (4) verbleibende Suspension unter weiterem Rühren auf die gewünschte Temperatur aufgeheizt. Dabei darf beim Einregeln der neuen, höheren Temperatur die Temperatur der Suspension nicht mehr als 3°C über die gewünschte Temperatur überschwingen. Ist die gewünschte Temperatur eingeregelt, wird bei konstanter Temperatur mindestens für eine Dauer von 4 Stunden nachgerührt.
(6) Nach dieser Nachrührzeit erfolgt erneut eine Probenahme, wie in Punkt (4) beschrieben. Bei jeder Probenahme wird generell ein neues Spritzenaufsatz-Filter verwendet. Liegt die gewünschte Temperatur mehr als 10°C über Raumtemperatur, wird die Spritze und das Spritzenaufsatz-Filter entsprechend vorgewärmt (z.B. in einem Wärmeschrank).
(7) Die gefilterte Flüssigkeitsprobe wird dann ebenfalls nach der in Punkt (4) beschriebenen Methode auf die Fumarsäure-Konzentration hin analysiert. Der analysierte Gehalt an Fumarsäure entspricht der Löslichkeit von Fumarsäure im eingesetzten Absorptionsmittel bei der eingeregelten Temperatur.
(8) Soll die Löslichkeit bei weiteren, noch höheren Temperaturen bestimmt werden, wird analog der Punkte (5) bis (7) verfahren. Dabei ist zu beachten, dass in zeitlicher Reihenfolge betrachtet die Fumarsäure-Löslichkeit aufgrund der ausgeprägten Neigung zur Übersättigung immer nur von der tieferen zur höheren Messtemperatur zu bestimmen ist.

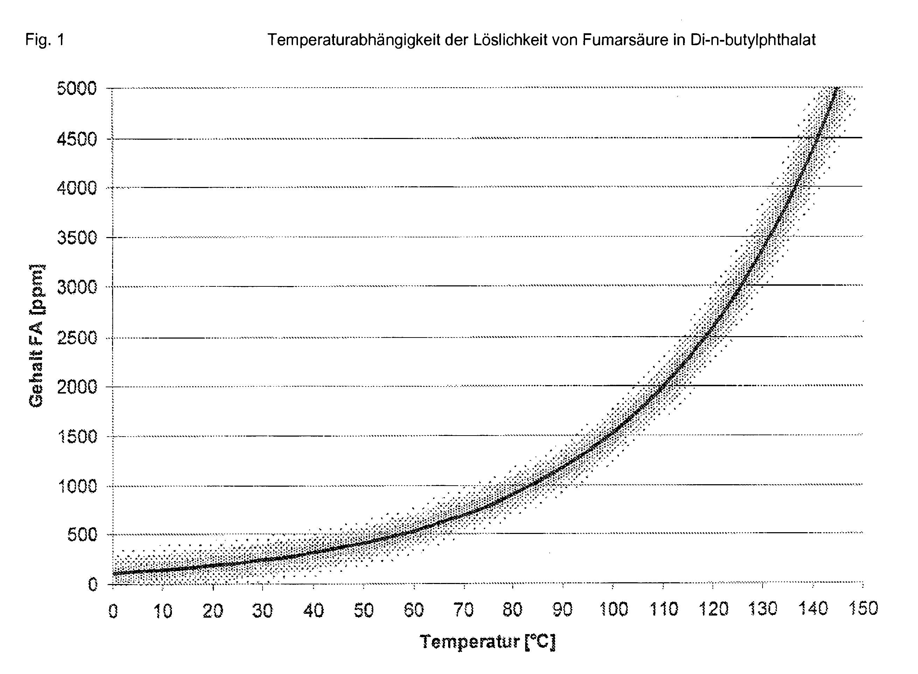

Die auf diese Art und Weise bestimmte Temperaturabhängigkeit der Löslichkeit von Fumarsäure in reinem Di-n-butylphthalat und in Di-n-butylphthalat basiertem Absorptionsmittel aus industriell betriebenen Anlagen ist in Fig. 1 vereinfacht dargestellt. Art und Menge der vorhandenen Nebenkomponenten bei den Di-n-butylphthalat basierten Absorptionsmitteln aus den industriell betriebenen Anlagen beeinflussen die Löslichkeit der Fumarsäure geringfügig. Als technisch bedingte Nebenkomponenten seien beispielhaft Wasser, Maleinsäureanhydrid, Maleinsäure, Acrylsäure, Methacrylsäure, Essigsäure, Propionsäure, Phthalsäureanhydrid und Phthalsäure genannt. Der grau hinterlegte Bereich in Fig. 1 gibt daher den Bereich an, in dem sich sowohl die Fumarsäure-Löslichkeit in reinem Di-n-butylphthalat als auch in den untersuchten Di-n-butylphthalat basierten Absorptionsmitteln aus industriell betriebenen Anlagen befindet. Die durchgezogene Linie entspricht der Ausgleichskurve.

Es sei betont, dass erfindungsgemäß natürlich immer die Fumarsäure-Löslichkeit im jeweils vorliegenden Absorptionsmittel zu berücksichtigen ist. Idealerweise ist daher bei technisch ausgeübten Verfahren auch die Temperaturabhängigkeit der Fumarsäure-Löslichkeit unter Einsatz einer Betriebsprobe aus der Absorptionsmittelrückführung zu ermitteln. Bei genauer, qualitativer und quantitativer Kenntnis der Nebenkomponenten ist es alternativ aber auch möglich, eine entsprechende synthetische Mischung einzusetzen.

Bei der Aufkonzentrierung durch Verdampfung eines Teils des Absorptionsmittels führt man die Gesamt- oder Teilmenge des in Schritt (b) von Maleinsäureanhydrid abgereicherten Absorptionsmittels im Allgemeinen einer unter Vakuum betriebenen Kolonne zu und zieht das verdampfte Absorptionsmittel über Kopf ab. Hierbei steigt formell die Konzentration an gelöster Fumarsäure und bei Überschreiten der Löslichkeit dann auch die Übersättigung. Um die Aufkonzentrierung zu fördern ist es in der Regel vorteilhaft, die Kolonne bei einer Temperatur oberhalb der des Absorptionsmittel-Rückführstroms zu betreiben und das aufkonzentrierte Absorptionsmittel anschließend wieder zu kühlen. Beim Einsatz des ganz besonders bevorzugten Di-n-butylphthalats lässt sich die Aufkonzentrierung besonders vorteilhaft bei einem Druck von 0,001 bis 0,004 MPa abs und einer Temperatur von 180bis 250°C durchführen.

Besonders vorteilhaft kühlt man in Schritt (d) des erfindungsgemäßen Verfahrens die Gesamt- oder Teilmenge des in Schritt (b) von Maleinsäureanhydrid abgereicherten Absorptionsmittels zur gezielten Ausfällung der Fumarsäure auf die niedrigste Temperatur in der Absorptionsmittelrückführung ab. Hierbei verringert sich die Löslichkeit der Fumarsäure im Absorptionsmittel und die Fumarsäure fällt teilweise in fester Form aus.

Das in Schritt (b) erhaltene, von Maleinsäureanhydrid abgereicherte Absorptionsmittel weist je nach Art der Abtrennung im Allgemeinen eine Temperatur von 100 bis 300°C auf. Es enthält im Allgemeinen 0,01 bis 5 Gew.-% und bevorzugt 0,02 bis 2 Gew.-% Fumarsäure und im Allgemeinen 0,01 bis 2 Gew.-% und bevorzugt 0,02 bis 0,5 Gew.-% Wasser. In der Regel ist der Gehalt an Fumarsäure umso höher, je höher der Gehalt an Wasser und je höher die Temperatur bei der Absorption in Schritt (a) und der Abtrennung in Schritt (b) ist. Neben der Fumarsäure enthält das abgereicherte Absorptionsmittel des Weiteren als Nebenprodukte auch Maleinsäure, alkylsubstituierte Maleinsäurederivate, Acrylsäure, Methacrylsäure, Essigsäure und Propionsäure. Hierzu kommen weitere Verbindungen, die sich aus dem Absorptionsmittel bilden können, wobei diese von der Natur des Absorptionsmittels abhängen. Werden beispielsweise Phthalsäureester (Phthalate) verwendet, so sind neben Phthalsäureanhydrid, Phthalsäure und deren Monoestern auch die durch Umesterung gebildeten Ester der vorstehend genannten Säuren möglich.

Die in Schritt (d) genannte, bevorzugte Abkühlung des in Schritt (b) erhaltenen, von Maleinsäureanhydrid abgereicherten Absorptionsmittels kann auf vielfältige Art und Weise erfolgen. Im Allgemeinen setzt man Kühlmedien ein, welche über eine Wärmetauscherfläche wirken. Als geeignete Kühlmedien seien beispielsweise Wasser, Luft oder andere gasförmige oder flüssige Ströme im Rahmen des Energieverbunds genannt. Bevorzugt ist dabei eine Abkühlung auf die niedrigste Temperatur in der Absorptionsmittelrückführung. Als Absorptionsmittelrückführung ist dabei der gesamte Bereich zwischen der Abtrennung des Maleinsäureanhydrids in Schritt (b) und der Absorption des Maleinsäureanhydrids in Schritt (a) zu verstehen, welcher von dem von Maleinsäureanhydrid abgereicherten Absorptionsmittel umspült wird. Um eine besonders vorteilhafte Abscheidungsrate der Fumarsäure im gewünschten lokalen Bereich in der Absorptionsmittelrückführung zu erzielen, kühlt man das Absorptionsmittel bei der gezielten Abkühlung bevorzugt um 1 bis 250°C, besonders bevorzugt um 50 bis 200°C und ganz besonders bevorzugt um 100 bis 150°C gegenüber den anderen Bereichen in der Absorptionsmittelrückführung ab. Die in Schritt (d) als niedrigste Temperatur bezeichnete Temperatur beträgt bevorzugt 10 bis 100°C, besonders bevorzugt 20 bis 90°C und ganz besonders bevorzugt 30 bis 70°C.

Bevorzugt kühlt man das an Maleinsäureanhydrid abgereicherte Absorptionsmittel soweit ab beziehungsweise konzentriert es durch Verdampfung von Absorptionsmittel soweit auf, dass die Menge an sich abscheidender Fumarsäure mindestens der Bildungsrate der Fumarsäure in der Gesamtanlage entspricht.

Der Druck des Absorptionsmittels in der Absorptionsmittelrückführung beträgt 0,01 bis 1 MPa abs, bevorzugt 0,09 bis 0,5 MPa abs und besonders bevorzugt 0,09 bis 0,3 MPa abs.

Beim erfindungsgemäßen Verfahren kann sowohl die Gesamtmenge als auch eine Teilmenge des in Schritt (b) von Maleinsäureanhydrid abgereicherten Absorptionsmittels in Schritt (d) zur gezielten Ausfällung der Fumarsäure abgekühlt und/oder durch Verdampfung eines Teils des Absorptionsmittels aufkonzentriert werden. Wird nur eine Teilmenge zur Ausfällung abgekühlt und/oder durch Verdampfung eines Teils des Absorptionsmittels aufkonzentriert, so wird der restliche Strom bevorzugt um die Ausfällungsstufe herumgeleitet und anschließend wieder mit dem an Fumarsäure abgereicherten Strom vereint oder alternativ getrennt zur Absorption in Schritt (a) zugeführt. Die Ausfällung aus einer Teilmenge kann gegenüber einer Ausfällung aus dem gesamten Strom besonders vorteilhaft sein. So kann aufgrund des geringeren Volumenstroms ein kleinerer und in der Regel auch preiswerterer Apparat eingesetzt werden. Des Weiteren kann beispielsweise bei der bevorzugten Abkühlung mit gleicher Kühlleistung eine niedrigere Temperatur erzielt und somit auch eine intensivere Abreicherung an der Fumarsäure in diesem Teilstrom erreicht werden. Insgesamt mag dies in Summe vorteilhafter sein als aus dem Gesamtstrom in einem größeren Apparat bei gleicher Kühlleistung eine weniger intensive Abreicherung der Fumarsäure zu erzielen. Analoges gilt entsprechend für die Aufkonzentrierung durch Verdampfung eines Teils des Absorptionsmittels.

Bevorzugt wird in Schritt (d) 5 bis 100% und besonders bevorzugt 50 bis 90% des in Schritt (b) von Maleinsäureanhydrid abgereicherten Absorptionsmittels abgekühlt und/oder durch Verdampfung eines Teils des Absorptionsmittels aufkonzentriert.

Durch die beschriebene Abkühlung beziehungsweise Aufkonzentrierung durch Verdampfung eines Teils des Absorptionsmittels fällt die Fumarsäure teilweise in fester Form aus, wobei die Ausfällung aufgrund der genannten ausgeprägten Neigung zur Übersättigung deutlich geringer ist als aufgrund der Löslichkeitskurve erwartet. Neben Fumarsäure fallen durch die Abkühlung gegebenenfalls noch weitere, weniger gut lösliche Nebenprodukte und Zersetzungsprodukte aus. Beim Einsatz von Phthalsäureestern als Absorptionsmittel insbesondere auch daraus gebildetes Phthalsäureanhydrid und Phthalsäure. Die Ausfällung kann kristalline oder amorphe Strukturen aufweisen. Dies kann bereits im Wärmetauscher oder danach geschehen. Die Ausfällung kann innerhalb der flüssigen Phase erfolgen oder auch als Ablagerung oder Aufwachsung an einer Rohr- oder Behälterwand. Durch Bereitstellung eine entsprechend großen Oberfläche, beispielsweise durch den Einsatz von Packungen oder Füllkörper, und/oder einer entsprechend langen Verweilzeit in dem Behälter, in dem die gezielte Ausfällung erfolgen soll, kann die Ausfällung besonders effektiv gestaltet werden. Dabei kann im Wesentlichen auf das allgemeine Fachwissen zur Kristallisation zurückgegriffen werden.

Die Ausfällung der Fumarsäure und der anderen Nebenkomponenten kann kontinuierlich oder diskontinuierlich erfolgen.

Um die Ausfällung der Fumarsäure aus dem abgekühlten beziehungsweise durch Verdampfung eines Teils des Absorptionsmittels aufkonzentrierten Strom weiter zu verbessern, ist es gegebenenfalls vorteilhaft, den abgekühlten beziehungsweise aufkonzentrierten Strom vor dessen Weiterleitung erst noch durch einen Verweilzeitbehälter zu leiten. Dieser sollte bevorzugt eine große innere Oberfläche aufweisen, um die Abscheidung der Fumarsäure weiter zu begünstigen. Geeignete Apparate hierzu sind beispielsweise mit Füllkörpern oder Packungen gefüllte Behälter. Diese können bei Bedarf kurzzeitig aus dem Strom genommen und gereinigt werden. Zur Reinigung kommen die weiter unten zur Entfernung der abgeschiedenen Fumarsäure beschriebenen Methoden in Frage.

In einer bevorzugten Ausführungsform setzt man zur Ausfällung und Abscheidung der Fumarsäure einen Behälter mit Einbauten ein. Aufgabe der Einbauten ist es insbesondere, eine entsprechende Oberfläche zur Abscheidung der ausgefallenen, also der bereits vorhandenen Fumarsäure-Partikel, beziehungsweise der ausfallenden Fumarsäure, also der aus dem noch gelösten Zustand auf der Oberfläche aufwachsenden Fumarsäure, bereitzustellen. Daher sind Einbauten mit einer hohen spezifischen Oberfläche besonders bevorzugt. Bevorzugt sollte dabei der Leervolumenanteil im Behälter bei 30 bis 99,5% und bevorzugt bei 90 bis 99% liegen. Die spezifische Oberfläche beträgt bevorzugt 50 bis 2000 m²/m³ und besonders bevorzugt 250 bis 1200 m²/m³. Hierfür können beispielsweise handelsübliche Schüttkörper, Packungen oder Drahtgestricke aus Stahl, Keramik, Porzellan oder Polymeren, bevorzugt aus nichtrostendem Stahl, verwendet werden. Die Oberfläche der Einbauten und Behälterwandungen kann glatt oder aufgerauht sein. Für die Einbauten kann auch Streckmetall oder Drahtgewebe verwendet werden. Die Einbaurichtung der Einbauten ist beliebig, bevorzugt jedoch horizontal oder vertikal. Werden Packungen verwendet, sollte der Knickwinkel der Kreuzkanalstrukturen um 10° bis 80°, bevorzugt um 40° bis 60° gegen die Durchströmungsrichtung geneigt sein.

Wird ein Abscheidevolumen mit Einbauten eingesetzt, so befindet sich dieses bevorzugt in dem Apparat, in dem die entsprechende Abkühlung und/oder Aufkonzentrierung durch Verdampfung eines Teils des Absorptionsmittels erfolgt, oder in Strömungsrichtung nachfolgend nach diesem Apparat.

Die mittlere Verweilzeit im Behälter mit Einbauten beträgt bevorzugt 0,05 bis 6 Stunden, besonders bevorzugt 0,1 bis 2 Stunden und ganz besonders bevorzugt 0,2 bis 1 Stunde. Die Durchströmungsgeschwindigkeit beträgt dabei bevorzugt 0,0005 bis 1,0 m/s und besonders bevorzugt, 0,001 bis 0,1 m/s. Die Behälter werden so betrieben, dass sich durch Abstimmung der Durchströmungsgeschwindigkeit und der spezifischen Oberfläche ein fluiddynamischer Zustand ergibt, bei dem sich eine Anlagerung von gefällten, suspendierten und/oder geflockten Teilchen an der inneren Oberfläche einstellt.

In einer besonderen Ausführungsform werden Einbauten mit unterschiedlicher spezifischer Oberfläche verwendet und so eingebaut, dass die spezifische Oberfläche in Strömungsrichtung zunimmt und sich die spezifische Oberfläche um den Faktor 1,5 bis 10, bevorzugt 2 bis 5, erhöht. In einer anderen besonderen Ausführungsform werden die Einbauten so angeordnet, dass sich zwischen den Einbauten Freiräume für die Zufuhr oder Entnahme von Flüssigkeiten befinden.

In einer anderen besonderen Ausführungsform kann es sich bei dem zuvor beschriebenen Behälter mit Einbauten auch um einen Teil der Absorptionseinheit aus Schritt (a) handeln, zu der gemäß Schritt (f) das abgekühlte und übersättigte Absorptionsmittel aus Schritt (d) ganz oder teilweise zu Schritt (a) rückgeführt wird. In diesem Falle würde die Absorptionseinheit, welche beispielsweise als sogenannte Absorptionskolonne ausgestaltet sein kann, im Bereich der Zufuhr des abgereicherten Absorptionsmittels bevorzugt Einbauten, wie in den oberen Absätzen betreffend den Behältern mit Einbauten beschrieben, aufweisen.

Die durch die Maßnahmen aus Schritt (d) als Feststoff ausgefallene Fumarsäure (sowie andere ausgefallene Nebenprodukte) wird dann in Schritt (e) ganz oder teilweise kontinuierlich oder diskontinuierlich aus der Absorptionsmittelrückführung entfernt. Im Allgemeinen entfernt man in Schritt (e) 5 bis 100%, bevorzugt 20 bis 100% und besonders bevorzugt 50 bis 100% der durch die Maßnahmen aus Schritt (d) als Feststoff ausgefallenen Fumarsäure. Bevorzugt setzt man dabei in Schritt (d) und (e) einen Apparat ein, aus dem man bei laufendem Durchsatz des von Maleinsäureanhydrid abgereicherten Absorptionsmittels die abgeschiedene Fumarsäure aus der Absorptionsmittelrückführung kontinuierlich oder diskontinuierlich entfernen kann. Im Falle von gezielt eingesetzten Einbauten in der Absorptionseinheit aus Schritt (a) erfolgt die Reinigung zweckmäßig bei den routinemäßigen Abstellungen der Anlage.

Die Entfernung der abgeschiedenen Fumarsäure kann auf unterschiedlichster Art und Weise erfolgen, beispielsweise mechanisch, physikalisch, thermisch oder chemisch. So kann die abgeschiedene Fumarsäure beispielsweise von der Oberfläche, auf der sie sich abgeschieden hat, mechanisch abgekratzt werden. Ferner ist es auch möglich, die abgeschiedene Fumarsäure nach Entleerung des Absorptionsmittels aus dem entsprechenden Behälter physikalisch zu lösen, beispielsweise in Wasser, bevorzugt in warmem oder heißem Wasser. Aufgrund der weniger guten Löslichkeit in Wasser ist es im Allgemeinen jedoch vorteilhafter, die abgeschiedene Fumarsäure chemisch in ein gut lösliches Salz zu überführen und dieses zu lösen. Dies geschieht beispielsweise durch Waschen mit einer wässrigen Base, bevorzugt Natronlauge. Ferner ist es auch möglich, die abgeschiedene Fumarsäure in Gegenwart von Sauerstoff thermisch abzubrennen. Falls die ausgefallene Fumarsäure im Absorptionsmittel suspendiert oder aufgeschlämmt vorliegt, kann diese beispielsweise durch Filter, Dekanter, Zyklone oder Zentrifugen abgetrennt werden. Besonders vorteilhaft sind, je nach Ausgestaltung des Apparats, in dem sich die Fumarsäure abscheidet, die Ausspülung durch Lösen in Natronlauge beziehungsweise die mechanische Entfernung durch Abkratzen.

Was die apparative Ausgestaltung der Ausfällung und Abscheidung der Fumarsäure durch die bevorzugte Abkühlung angeht, sind im Wesentlichen drei grundsätzliche Prinzipien von besonderer Bedeutung, die natürlich auch kombiniert werden können. Sie werden im Folgenden näher erläutert.
A) Bei der ersten apparativen Ausgestaltung setzt man in Schritt (d) und (e) einen Apparat mit mindestens zwei parallelen Ausfällungszonen ein, aus dem man die abgeschiedene Fumarsäure bei laufendem Durchsatz des von Maleinsäureanhydrid abgereicherten Absorptionsmittels durch mindestens eine der Ausfällungszonen aus mindestens einer anderen Ausfällungszonen diskontinuierlich entfernen kann.
   Die einfachste Form hierzu ist der Einsatz von genau zwei parallelen Ausfällungszonen. Im Fachjargon spricht man auch von einer A/B-Ausführung. Hier kann beispielsweise zunächst das an Fumarsäure abzureichernde Absorptionsmittel nur über Ausfällungszone A geleitet werden, in dem es sich abkühlt und die Fumarsäure ausfällt. Hat sich in Ausfällungszone A genug Fumarsäure abgeschieden, kann dann auf Ausfällungszone B umgeschaltet werden und die abgeschiedene Fumarsäure dann aus Ausfällungszone A entfernt werden. Hat sich dann in Ausfällungszone B genug Fumarsäure abgeschieden, wird wieder auf den betriebsbereiten Ausfällungszone A umgeschaltet.
   Alternativ können auch die beiden Ausfällungszonen A und B parallel zur Abscheidung von Fumarsäure eingesetzt werden. Hat sich in einem der beiden Ausfällungszonen dann genug Fumarsäure abgeschieden, so kann diese nach Umschaltung auf die anderen Ausfällungszonen entfernt werden. Anschließend kann dann die gereinigte Ausfällungszone wieder parallel zugeschaltet werden. Vorteilhafterweise werden die beiden Ausfällungszonen natürlich so betrieben, dass deren Zyklen entsprechend versetzt liegen.
   Entsprechend der obigen Beschreibung einer A/B-Ausführung ist natürlich auch der Einsatz von mehr als zwei parallelen Ausfällungszonen (nachfolgend n Ausfällungszonen genannt), möglich. Bevorzugt werden dann n-1 Ausfällungszonen oder alle n Ausfällungszonen parallel zur Abscheidung der Fumarsäure genutzt. Hat sich in einer der genutzten Ausfällungszonen genug Fumarsäure abgeschieden, so kann diese zu deren Entfernung abgeschaltet werden. Falls eine betriebsbereite, nicht benutzte Ausfällungszone vorhanden ist, kann diese dann zugeschaltet werden. Nach der Reinigung der abgeschalteten Ausfällungszone wird diese dann entweder sofort wieder zugeschaltet oder in Warteposition gehalten bis eine andere Ausfällungszone abgeschaltet wird. Vorteilhafterweise werden auch bei mehr als zwei Ausfällungszonen diese natürlich so betrieben, dass deren Zyklen entsprechend versetzt liegen.
   Der besondere Vorteil der ersten apparativen Ausgestaltung liegt darin, dass das Verfahren ohne Unterbrechung der gezielten Ausfällung der Fumarsäure betrieben werden kann. Nachteilig daran ist, dass mindestens zwei parallele Ausfällungszonen erforderlich sind. Dieser Nachteil wird jedoch nahezu bedeutungslos, wenn aus anlagentechnischen Gründen sowieso schon parallele Einrichtungen vorhanden oder einzusetzen sind. Als bevorzugte Ausführungsform hierzu sei der Einsatz eines Luftkühlers mit mehreren parallelen Registern genannt.
B) Bei der zweiten apparativen Ausgestaltung setzt man in Schritt (d) und (e) einen Apparat mit Bypass ein, aus dem man die abgeschiedene Fumarsäure bei laufendem Durchsatz des von Maleinsäureanhydrid abgereicherten Absorptionsmittels durch den Bypass aus dem Apparat diskontinuierlich entfernen kann.
   Bei dieser Variante leitet man das an Fumarsäure abzureichernde Absorptionsmittel durch die Ausfällungszone des Apparats. Wurde in dieser genug Fumarsäure abgeschieden, schaltet man den Apparat zur Reinigung ab und leitet das Absorptionsmittel über den Bypass an diesem Apparat vorbei. Anschließend schaltet man den gereinigten Apparat wieder zu.
   Alternativ ist es auch möglich, stets einen Teil des an Fumarsäure abzureichernde Absorptionsmittels durch den Bypass zu leiten. Der zur Ausfällung bestimmte Apparat wird somit nur mit einem geringeren Flüssigkeitsstrom belastet und kann daher auch kleiner ausgelegt werden. Mit gleicher Kühlleistung ist aufgrund des geringeren Absorptionsmittelstroms eine niedrigere Temperatur erreichbar, was zu einer höheren, relativen Abscheidung von Fumarsäure führt. Ist der Apparat zu reinigen, so wird kurzzeitig der gesamte Strom über den Bypass geleitet.
   In einer besonderen Bauform kann die Aufällungszone auch in einen der vorhandenen Apparate, bevorzugt den Apparat für die Maleinsäureanhydrid-Absorption, integriert werden.
   Der besondere Vorteil der zweiten apparativen Ausgestaltung liegt darin, dass nur ein Apparat mit einer Ausfällungszone erforderlich ist. Nachteilig daran ist jedoch, dass während der Reinigung des Apparats keine gezielte Ausfällung der Fumarsäure erfolgen kann. Durch eine schnelle und unkomplizierte Reinigungsprozedur des abgeschalteten Apparats kann dieser Nachteil jedoch stark gemindert werden.
C) Bei der dritten apparativen Ausgestaltung setzt man in Schritt (d) und (e) einen Apparat ein, aus dem man bei laufendem Durchsatz des von Maleinsäureanhydrid abgereicherten Absorptionsmittels dieabgeschiedene Fumarsäure bei fortlaufendem Betrieb der Ausfällungszone aus der Absorptionsmittelrückführung kontinuierlich oder diskontinuierlich entfernen kann.
   Hierunter fallen beispielsweise Apparate, welche die an einer Oberflächen abgeschiedene Fumarsäure bei laufendem Betrieb kontinuierlich oder diskontinuierlich abkratzen und abführen, wie dies zum Beispiel bei den bekannten, handelsüblichen Kühl- und Walzenkristallern der Fall ist. Des Weiteren fallen hierunter auch Apparate, in welchen die ausgefallene Fumarsäure als Suspension oder Aufschlämmung anfällt, aus der sie dann durch mechanisch-physikalische Methoden, wie etwa durch Filter, Dekanter, Zyklone oder Zentrifugen, abgetrennt werden kann.
   Der besondere Vorteil der dritten apparativen Ausgestaltung liegt darin, dass nur ein Apparat mit einer Ausfällungszone erforderlich ist und dieser kontinuierlich ohne Abschaltung und separate Reinigung betrieben werden kann. Nachteilig daran ist jedoch der etwas erhöhte apparative Aufwand durch den Einsatz besonderer Apparate wie beispielsweise eines Kristallers, Filters, Dekanters, Zyklons oder einer Zentrifuge.

Bei der Wahl der apparativen Ausgestaltung der Ausfällung der Fumarsäure ist es besonders vorteilhaft, die genannten Vor- und Nachteil unter Berücksichtigung des Gesamtsystems gegeneinander abzuwägen.

Das an Fumarsäure abgereicherte Absorptionsmittel aus Schritt (d) führt man in Schritt (f) ganz oder teilweise zu Schritt (a) zurück. Im Allgemeinen führt man in Schritt (f) 10 bis 100%, bevorzugt 50 bis 100% und besonders bevorzugt 90 bis 100% des an Fumarsäure abgereicherten Absorptionsmittels aus Schritt (d) zu Schritt (a) zurück. Im Hinblick auf die Ausfällung von Fumarsäure kann es im Allgemeinen ohne nachgeschaltete Aufheizung zu Schritt (a) zurückgeführt werden, da die infolge der Übersättigung in der technisch relevanten Zeitskala ausfällbare Fumarsäure in der Regel bereits ausgefallen ist. Bei Bedarf, beispielsweise zur Erreichung der gewünschten Eintrittstemperatur für die Aborptionskolonne, steht einer Aufheizung jedoch nichts entgegen.

Das beim erfindungsgemäßen Verfahren in Schritt (a) einzusetzende, Maleinsäureanhydrid enthaltende Rohproduktgemisch kann in einer vorgeschalteten Stufe durch heterogenkatalytische Oxidation eines Kohlenwasserstoffs ausgewählt aus der Gruppe Benzol, n-Butan, n-Buten und 1,3-Butadien, mit molekularem Sauerstoff in Gegenwart eines Vanadium, Phosphor und Sauerstoff enthaltenden Katalysators gewonnen werden. Im Allgemeinen führt man die heterogenkatalytische Oxidation in einem Rohrbündelreaktor durch. Verfahren zur Oxidation von n-Butan sind beispielsweise in Ullmann's Encyclopedia of Industrial Chemistry, 2005 Wiley-VCH Verlag GmbH & Co KGaA, Weinheim, "Maleic and Fumaric Acids - Maleic Anhydride" beschrieben.

Das so erhaltene Rohproduktgemisch wird dann in Schritt (a) in einem geeigneten organischen Lösungsmittel als Absorptionsmittel aufgenommen.

Das Maleinsäureanhydrid enthaltende Rohproduktgemisch kann in vielfältiger Weise, bevorzugt bei Drucken von 0,08 bis 1 MPa abs und einer Temperatur von 50 bis 300°C in einer oder mehreren Absorptionsstufen mit dem Lösungsmittel (Absorptionsmittel) in Kontakt gebracht werden: (i) durch Einleiten des Gasstroms in das Lösungsmittel (z.B. über Gaseinleitungsdüsen oder Begasungsringe), (ii) durch Einsprühen des Lösungsmittels in den Gasstrom oder (iii) durch Gegenstromkontakt zwischen dem nach oben strömenden Gasstrom und dem nach unten strömenden Lösungsmittel in einer Boden- oder Packungskolonne. In allen drei Varianten können die dem Fachmann bekannten Apparate zur Gasabsorption eingesetzt werden. Bei der Wahl des einzusetzenden Lösungsmittels (Absorptionsmittel) ist darauf zu achten, dass dieses nicht mit dem Edukt, dem eingesetzten Maleinsäureanhydrid, reagiert. Zudem ist aufgrund der nachfolgenden Abtrennung des Maleinsäureanhydrids vom Absorptionsmittel auf einen entsprechenden Unterschied in den Siedepunkten des Absorptionsmittels und des Maleinsäureanhydrids zu achten. Bevorzugt hat das organische Lösungsmittel, auf Atmosphärendruck bezogen, einen um mindestens 30°C höheren Siedepunkt als das Maleinsäureanhydrid.

Geeignete Absorptionsmittel sind beispielsweise Phosphorsäureester (z.B. Trikresylphosphat), Maleinsäureester (z.B. Dibutylmaleat, Butylmaleat), hochmolekulare Wachse, aromatische Kohlenwasserstoffe mit einem Molekulargewicht zwischen 150 und 400 g/Mol und einem Siedepunkt oberhalb 140°C (z.B. Dibenzylbenzol), Phthalsäureester (z.B. Alkylphthalate und Dialkylphthalate mit C₁-C₁₈-Alkylgruppen, beispielsweise Dimethylphthalat, Diethylphthalat, Di-n-propylphthalat, Di-iso-propylphthalat, Di-n-butylphthalat, Diundecylphthalat, Methylphthalat, Ethylphthalat, n-Propylphthalat, iso-Propylphthalat, Butylphthalat, Undecylphthalat), Di-C₁-C₄-Alkylester anderer aromatischer und aliphatischer Dicarbonsäuren (z.B. Dimethyl-2,3-naphthalin-dicarbonsäure-dimethylester, Dimethyl-1,4-cyclohexan-dicarbonsäure-dimethylester), C₁-C₄-Alkylester anderer aromatischer und aliphatischer Dicarbonsäuren (z.B. 2,3-Naphthalin-dicarbonsäure-dimethylester, 1,4-Cyclohexan-dicarbonsäure-dimethylester langkettiger Fettsäuren mit beispielsweise 14 bis 30 Kohlenstoffatomen), hochsiedende Ether (z.B. Dimethylether von Polyethylenglykol oder Tetraethylenglykoldimethylether).

Bevorzugt setzt man Phthalsäureester, besonders bevorzugt Di-(C₁- bis C₁₂-alkyl)-phthalat und ganz besonders bevorzugt Di-n-butylphthalat ein.

Die durch die Absorption in Schritt (a) resultierende Lösung hat im Allgemeinen einen Gehalt an Maleinsäureanhydrid von etwa 5 bis 400 g/L.

Der nach der Absorption in Schritt (a) verbleibende Abgasstrom enthält neben Wasser hauptsächlich die Nebenprodukte der vorangegangenen Oxidation, wie etwa Kohlenmonoxid, Kohlendioxid, nicht umgesetzte Ausgangs-Kohlenwasserstoffe sowie Essig- und Acrylsäure. Der Abgasstrom ist weitgehend frei von Maleinsäureanhydrid. Anschließend trennt man in Schritt (b) das Maleinsäureanhydrid von dem in Schritt (a) erhaltenen, mit Maleinsäureanhydrid angereicherten Absorptionsmittel ab. Die Abtrennung erfolgt bevorzugt durch Strippung mit einem geeigneten Gas, insbesondere Wasserstoff, oder durch Destillation.

Eine Strippung mit Wasserstoff ist vor allem dann von Vorteil, wenn das Maleinsäureanhydrid anschließend zu Tetrahydrofuran, 1,4-Butandiol und/oder gamma-Butyrolacton hydriert werden soll. In diesem Fall erfolgt die Strippung bevorzugt bei einer Temperatur von 100 bis 250°C und einem Druck von 0,08 bis 3 MPa abs, wobei der Druck bevorzugt maximal 10% oberhalb des Druckes der sich anschließenden Hydrierung liegt. In der Strippkolonne beobachtet man in der Regel ein Temperaturprofil, das sich aus den Siedepunkten von Maleinsäureanhydrid am Kopf und dem nahezu Maleinsäureanhydrid-freien Absorptionsmittel im Sumpf der Kolonne bei dem jeweiligen Kolonnendruck und der eingestellten Verdünnung mit Trägergas (im ersten Fall mit Wasserstoff) ergibt. Um Verluste an Absorptionsmittel zu verhindern, können sich oberhalb der Zufuhr des mit Maleinsäureanhydrid angereicherten Absorptionsmittels Rektifiziereinbauten befinden.

Alternativ zur Wasserstoffstrippung kann man das im Absorptionsmittel gelöste Maleinsäureanhydrid auch in einer Destillationseinheit bei Drücken von im Allgemeinen 0,001 bis 0,5 MPa abs und Temperaturen von 65 bis 300°C abtrennen. Die Destillation kann dabei in einer Stufe oder mehreren Stufen, beispielsweise in Trennapparaturen mit einer Stufe oder mehreren Stufen, wie zum Beispiel Kolonnen mit mehreren Trennstufen, beispielsweise Rektifikationskolonnen, Füllkörperkolonnen, Glockenbödenkolonnen oder Packungskolonnen durchgeführt werden.

Bevorzgt führt man in Schritt (c) 50 bis 100% und besonders bevorzugt 90 bis 100% des in Schritt (b) von Maleinsäureanhydrid abgereicherten Absorptionsmittels zu Schritt (a) zurück.

In einer grundlegenden Verfahrensweise wird das von Maleinsäureanhydrid befreite Absorptionsmittel zunächst entgast und im Folgenden durch einen Luftkühler auf eine leicht über der in der folgenden Verfahrensstufe der Absorption benötigten Temperatur abgekühlt. Diese Temperatur wird so gewählt, dass eine Abscheidung der im Absorptionsmittel angereicherten Fumarsäure gerade noch nicht erfolgt. In einer nachfolgenden Kühlstufe, die als Luft- oder Wasserkühler ausgeführt sein kann, wird nun unter Abscheidung von Fumarsäure auf eine Temperatur abgekühlt, welche zu einer Differenz zwischen der Konzentration an Fumarsäure im Rückführstrom am Ausgang der Desorptionskolonne c(FA, Ausgang Desorptionskolonne) unter den dort vorliegenden Bedingungen in Gew.-ppm und der Gleichgewichtskonzentration an Fumarsäure gemäß der Löslichkeitskurve nach der Abkühlung c(FA, Gleichgewicht nach Abkühlung) in Gew.-ppm von größer oder gleich 250 Gew.-ppm führt. Hierdurch kann die Ausfällung von Fumarsäure so gesteuert werden, dass nur dieser Apparat in regelmäßigen Abständen gereinigt werden muss. Eine Außerbetriebsetzung der Gesamtanlage kann durch Ausführung dieses Kühlers in A/B-Schaltung vermieden werden.

Wird nur ein Teilstrom durch den Abscheidekühler geleitet, so kann dieser weiter verkleinert werden. Außerdem kann so für das kurze Abreinigungsintervall die Kristallisationsstufe einfach umfahren werden und so eine A/B-Ausführung vermieden werden. Der Kühler selbst kann auch hier als singulärer Kühler oder, wesentlich effizienter, als Verschaltung von Kreuzstromkühler und Abscheidekühler wie im vorangehenden Absatz beschrieben, ausgeführt werden.

Bei bestehenden Anlagen, bei denen der Hauptkühler aus mehreren parallelen Strängen besteht, wie es bei Luftkühlern in Form einzelner Register der Fall ist, kann durch Androsseln einzelner Register eine ebenfalls sehr einfache und effiziente Abscheidung der Fumarsäure erreicht werden: Das angedrosselte Register kühlt sich deutlich stärker ab und scheidet die Fumarsäure sehr effektiv ab, während das wärmere Hauptregister weitgehend belagsfrei bleibt. Das Opferregister kann auch hier nach Abtrennung mit Schiebern in den Zu- und Ableitungen während des Betriebes des Hauptregisters ohne Unterbrechung des Prozesses abgereichert werden.

Das erfindungsgemäße Verfahren ermöglicht die deutliche Verringerung von Fumarsäureablagerungen auf Anlagenteilen und dadurch verursachte Verstopfungen, Ausbau- und Reinigungsarbeiten sowie Abschaltungen bei der Herstellung von Maleinsäureanhydrid, wobei das Verfahren mit relativ geringem technischen Aufwand durchgeführt werden kann und die bekannten Nachteile aus dem Stand der Technik vermieden werden.

### Beispiele

### Beispiel 1 (erfindungsgemäß)

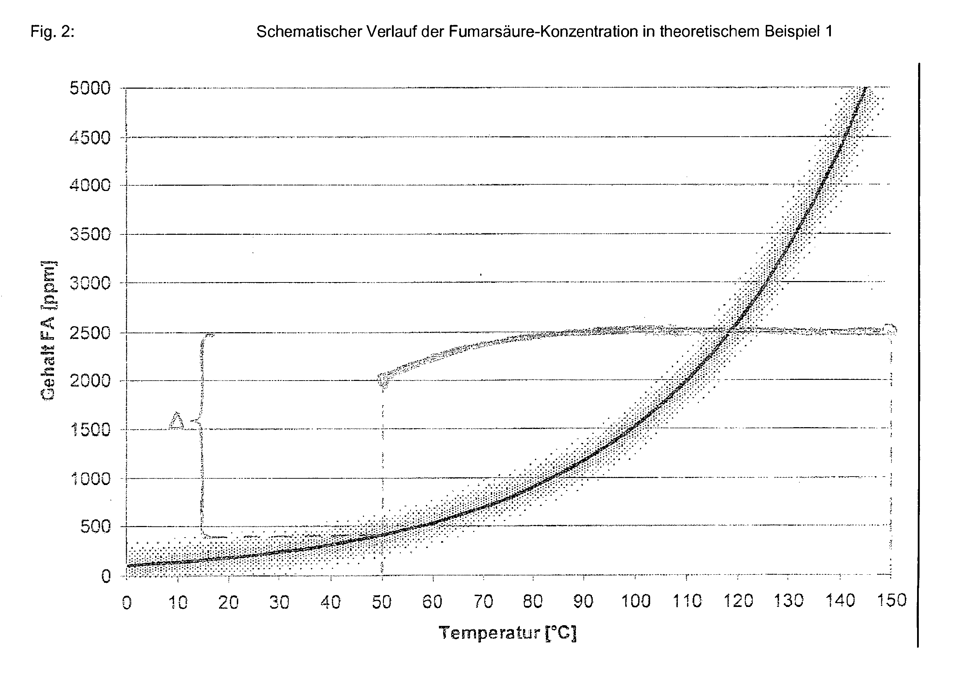

In einem theoretischen Beispiel wird das an Maleinsäureanhydrid abgereicherte Di-n-butylphthalat bei 150°C aus dem Sumpf der Desorptionskolonne entnommen. Die Konzentration an Fumarsäure betrage 2500 Gew.-ppm. Der Rückführstrom wird vollständig in einen Luftkühler mit zehn identischen Registern geleitet. 50% des 150°C warmen Stroms werden durch zwei Register geleitet und darin auf 100°C abgekühlt. Die anderen 50% werden durch vier Register geleitet und auf 50°C abgekühlt. Dabei scheidet sich ein Teil der Fumarsäure in den vier Registern ab und die Konzentration an gelöster Fumarsäure (ermittelt durch Probenahme unter Verwendung der bereits beschriebenen 0,2 Mikrometer-Membranfilter) sinkt auf 2000 Gew.-ppm. Anschließend werden beide Ströme zu einem Strom mit 75°C und 2250 Gew.-ppm gelöster Fumarsäure vereint und der Absorptionskolonne zugeführt. Der Verlauf der Fumarsäure-Konentration in dem durch die vier Register geleiteten Stroms ist schematisch in Fig. 2 dargestellt. Die Gleichgewichtskonzentration an Fumarsäure gemäß der Löslichkeitskurve nach der Abkühlung auf 50°C c(FA, Gleichgewicht nach Abkühlung/Verdampfung) beträgt etwa 400 Gew.-ppm. Somit beträgt die Differenz c(FA, Ausgang Desorptionskolonne) minus c(FA, Gleichgewicht nach Abkühlung/Verdampfung) etwa 2100 Gew.-ppm.

Nachdem sich in den zur Abkühlung auf 50°C verwendeten vier Registern der Strömungswiderstand aufgrund der Fumarsäure-Ablagerungen merklich erhöht hat, wird bei laufendem Betrieb auf die anderen vier Register umgeschaltet und die mit Fumarsäure belegten Register mit wässriger Natronlauge oder heißem Wasser gereinigt.

### Experimentelle Versuchsdurchführung für die Beispiele 2 bis 5

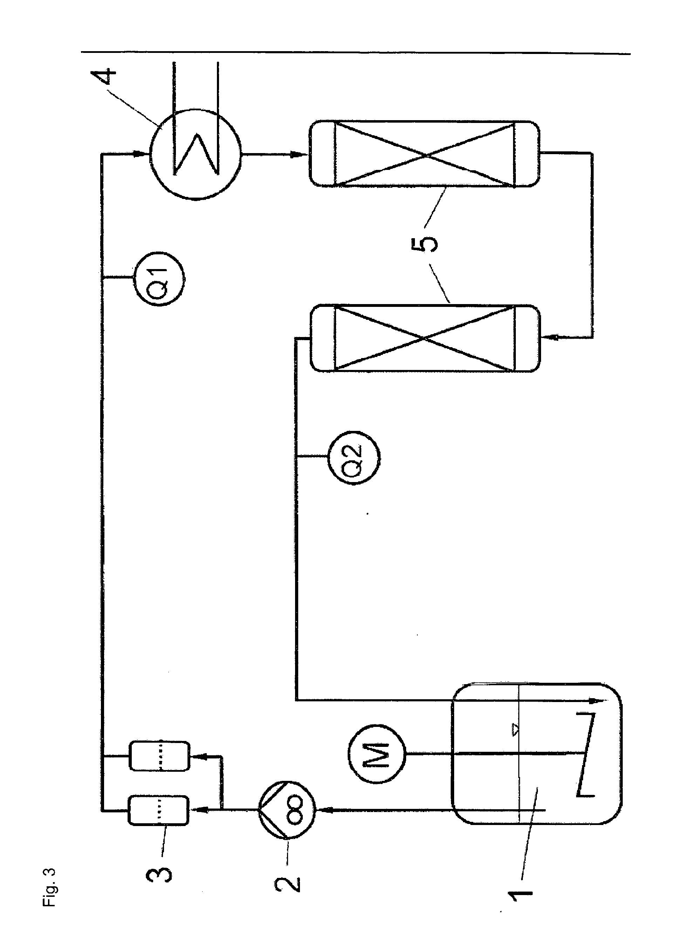

Für die Durchführung der experimentellen Beispiele 2 bis 5 wurde eine Versuchsanlage im Labormaßstab eingesetzt. Fig. 3 zeigte eine vereinfachte Darstellung dieser Versuchsanlage. Dibutylphtalat wurde in einem Rührbehälter (1), welcher ein Füllvolumen von 8 L aufwies, bei einer Temperatur von 95°C bis 120°C mit Fumarsäure angereichert, mit einer Pumpe (2) über Filter (3) in einem Kühler (4) auf 30°C bis 70°C abgekühlt und durch eine Verweilzeitstrecke (5) geleitet. Die Verweilzeitstrecke bestand aus 2 Glasrohren mit einem Innendurchmesser von 30 mm. Die Glasrohre waren jeweils mit Packungen (Kühni Rombopak 9M) gefüllt, Packungshöhe 2 x 1 m. Rührbehälter und Glasrohre wurden zur Temperierung doppelwandig ausgeführt. Nach der Verweilzeitstrecke wurde die Lösung wieder dem Rührbehälter zugeführt und mit Fumarsäure angereichert. In der Verweilzeitstrecke wurde die Fumarsäure bei Einstellen einer erfindungsgemäßen Übersättigung teilweise abgeschieden. Die Probenahme zur Bestimmung der Fumarsäurekonzentration erfolgte vor dem Kühler (Q1) und nach der Verweilzeitstrecke (Q2). Im Rührbehälter war immer soviel Fumarsäure vorhanden, dass ein Bodenkörper verblieb.

### Beispiel 2 (erfindungsgemäß)

Der Versuch wurde mit einer Mischung aus Dibutylphtalat und Fumarsäure durchgeführt. Die Sättigungskonzentration von Fumarsäure in der Lösung bei 50°C betrug 250 Gew.-ppm. In der Versuchsanlage wurde ein Volumenstrom von 15,7 L/h eingestellt, das entspricht einer Verweilzeit von 0,09 h bzw. einer Strömungsgeschwindigkeit von 0,00617 m/s in der Verweilzeitstrecke. Im Rührbehälter wurde eine Temperatur von 100°C eingestellt, am Ausgang des Kühlers 50°C. Vor dem Kühler wurde eine Fumarsäurekonzentration von 657 Gew.-ppm gemessen. Die Konzentrationsdifferenz c(vor Kühler) - c(Sättigung bei 50°C nach Kühler) betrug damit 407 Gew.-ppm. Die Fumarsäurekonzentration nach der Verweilzeitstrecke betrug 359 Gew.-ppm. In der Verweilzeitstrecke wurden damit 298 Gew.-ppm abgeschieden.

### Beispiel 3 (Vergleichsbeispiel)

Der Versuch wurde mit einer Mischung aus Dibutylphtalat und Fumarsäure durchgeführt. Die Sättigungskonzentration von Fumarsäure in der Lösung bei 50°C betrug 250 Gew.-ppm. In der Versuchsanlage wurde ein Volumenstrom von 15,7 L/h eingestellt, das entspricht einer Verweilzeit von 0,09 h bzw. einer Strömungsgeschwindigkeit von 0,00617 m/s in der Verweilzeitstrecke. Im Rührbehälter wurde eine Temperatur von 115°C eingestellt, am Ausgang des Kühlers 50°C. Vor dem Kühler wurde eine Fumarsäurekonzentration von 435 Gew.-ppm ermittelt. Die Konzentrationsdifferenz c(vor Kühler) - c(Sättigung bei 50°C nach Kühler) betrug damit 185 Gew.-ppm. Die Fumarsäurekonzentration nach der Verweilzeitstrecke wurde zu 416 Gew.-ppm ermittelt.

Unter Berücksichtigung der Mess- bzw. Analysegenauigkeit zeigt dieses Beispiel, dass es unter den eingestellten Bedingungen zu keiner Abscheidung von Fumarsäure in der Verweilzeitstrecke kommt.

### Beispiel 4 (erfindungsgemäß)

Der Versuch wurde mit einer Lösung aus einer industriell betriebenen Anlage zur Herstellung von Maleinsäureanhydrid (Dibutylphtalat-Konzentration >98,5 Gew.-%), durchgeführt. Die Sättigungskonzentration von Fumarsäure in der Lösung bei 30°C betrug 250 Gew.-ppm. In der Versuchsanlage wurde ein Volumenstrom von 3,5 L/h eingestellt, das entspricht einer Verweilzeit von 0,404 h bzw. einer Strömungsgeschwindigkeit von 0,00138 m/s in der Verweilzeitstrecke. Im Rührbehälter wurde eine Temperatur von 120°C eingestellt, am Ausgang des Kühlers 30°C. Vor dem Kühler wurde eine Fumarsäurekonzentration von 1043 Gew.-ppm gemessen. Die Konzentrationsdifferenz c(vor Kühler) - c(Sättigung bei 30°C nach Kühler) betrug damit 793 Gew.-ppm. Die Fumarsäurekonzentration nach der Verweilzeitstrecke betrug 634 Gew.-ppm. In der Verweilzeitstrecke wurden damit 409 Gew.-ppm abgeschieden.

### Beispiel 5 (erfindungsgemäß)

Der Versuch wurde mit einer Lösung aus einer industriell betriebenen Anlage zur Herstellung von Maleinsäureanhydrid (Dibutylphtalat-Konzentration >98,5 Gew.-%) durchgeführt. Die Sättigungskonzentration von Fumarsäure in der Lösung bei 50°C betrug 400 Gew.-ppm. In der Versuchsanlage wurde ein Volumenstrom von 3,5 L/h eingestellt, das entspricht einer Verweilzeit von 0,404 h bzw. einer Strömungsgeschwindigkeit von 0,00138 m/s in der Verweilzeitstrecke. Im Rührbehälter wurde eine Temperatur von 95°C eingestellt, am Ausgang des Kühlers 50°C. Vor dem Kühler wurde eine Fumarsäurekonzentration von 1130 Gew.-ppm gemessen. Die Konzentrationsdifferenz c(vor Kühler) - c(Sättigung bei 50°C nach Kühler) betrug damit 730 Gew.-ppm. Die Fumarsäurekonzentration nach der Verweilzeitstrecke betrug 1059 Gew.-ppm. In der Verweilzeitstrecke wurden damit 71 Gew.-ppm abgeschieden.

## Patentansprüche

1. Verfahren zur Verringerung von Fumarsäureablagerungen bei der Herstellung von Maleinsäureanhydrid durch heterogenkatalytische Oxidation eines Kohlenwasserstoffs ausgewählt aus der Gruppe Benzol, n-Butan, n-Buten und 1,3-Butadien, mit molekularem Sauerstoff in Gegenwart eines Vanadium, Phosphor und Sauerstoff enthaltenden Katalysators, umfassend
(a) die Absorption von Maleinsäureanhydrid aus dem Rohproduktgemisch in einem Absorptionsmittel enthaltend ein organisches Lösungsmittel in einer Absorptionskolonne;
(b) die Desorption des Maleinsäureanhydrids von dem in Schritt (a) erhaltenen, mit Maleinsäureanhydrid angereicherten Absorptionsmittel in einer Desorptionskolonne; und
(c) die vollständige oder teilweise Rückführung des in Schritt (b) von Maleinsäureanhydrid abgereicherten Absorptionsmittels zu Schritt (a),
**dadurch gekennzeichnet, dass** man
(d) die Gesamt- oder Teilmenge des in Schritt (b) von Maleinsäureanhydrid abgereicherten Absorptionsmittels zur gezielten Ausfällung der Fumarsäure soweit abkühlt und/oder durch Verdampfung eines Teils des Absorptionsmittels aufkonzentriert, dass die Differenz zwischen der Konzentration an Fumarsäure im Rückführstrom am Ausgang der Desorptionskolonne c(FA, Ausgang Desorptionskolonne) unter den dort vorliegenden Bedingungen in Gew.-ppm und der Gleichgewichtskonzentration an Fumarsäure gemäß der Löslichkeitskurve nach der Abkühlung und/oder Verdampfung eines Teils des Absorptionsmittels c(FA, Gleichgewicht nach Abkühlung/Verdampfung) in Gew.-ppm größer oder gleich 250 Gew.-ppm beträgt und man zur Ausfällung und Abscheidung der Fumarsäure einen Behälter mit Einbauten einsetzt, wobei die mittlere Verweilzeit im Behälter mit Einbauten 0,05 bis 6 Stunden beträgt;
(e) die durch die Maßnahmen aus Schritt (d) als Feststoff ausgefallene Fumarsäure ganz oder teilweise kontinuierlich oder diskontinuierlich aus der Absorptionsmittelrückführung entfernt; und
(f) das an Fumarsäure abgereicherte Absorptionsmittel aus Schritt (e) ganz oder teilweise zu Schritt (a) rückführt.

2. Verfahren nach Anspruch 1, **dadurch gekennzeichnet, dass** in Schritt (d) die mittlere Verweilzeit im Behälter mit Einbauten mindestens 0,1 Stunde beträgt.

3. Verfahren nach den Ansprüchen 1 bis 2, **dadurch gekennzeichnet, dass** man in Schritt (d) die Gesamt- oder Teilmenge des in Schritt (b) von Maleinsäureanhydrid abgereicherten Absorptionsmittels zur gezielten Ausfällung der Fumarsäure soweit abkühlt und/oder durch Verdampfung eines Teils des Absorptionsmittels aufkonzentriert, dass die Differenz c(FA, Ausgang Desorptionskolonne) - c(FA, Gleichgewicht nach Abkühlung/Verdampfung) größer oder gleich 500 Gew.-ppm beträgt.

4. Verfahren nach den Ansprüchen 1 bis 3, **dadurch gekennzeichnet, dass** man in Schritt (d) auf die niedrigste Temperatur in der gesamten Absorptionsmittelrückführung abkühlt.

5. Verfahren nach den Ansprüchen 1 bis 4, **dadurch gekennzeichnet, dass** man in Schritt (d) auf eine Temperatur im Bereich 10 bis 100°C abkühlt.

6. Verfahren nach den Ansprüchen 1 bis 5, **dadurch gekennzeichnet, dass** man in Schritt (d) und (e) einen Apparat einsetzt, aus dem man bei laufendem Durchsatz des von Maleinsäureanhydrid abgereicherten Absorptionsmittels die abgeschiedene Fumarsäure aus der Absorptionsmittelrückführung kontinuierlich oder diskontinuierlich entfernen kann.

7. Verfahren nach den Ansprüchen 1 bis 6, **dadurch gekennzeichnet, dass** man in Schritt (d) und (e) einen Apparat mit mindestens zwei parallelen Ausfällungszonen einsetzt, aus dem man die abgeschiedene Fumarsäure bei laufendem Durchsatz des von Maleinsäureanhydrid abgereicherten Absorptionsmittels durch mindestens eine der Ausfällungszonen aus mindestens einer anderen Ausfällungszonen diskontinuierlich entfernen kann.

8. Verfahren nach den Ansprüchen 1 bis 7, **dadurch gekennzeichnet, dass** man in Schritt (d) und (e) einen Apparat mit Bypass einsetzt, aus dem man die abgeschiedene Fumarsäure bei laufendem Durchsatz des von Maleinsäureanhydrid abgereicherten Absorptionsmittels durch den Bypass aus dem Apparat diskontinuierlich entfernen kann.

9. Verfahren nach den Ansprüchen 1 bis 8, **dadurch gekennzeichnet, dass** man in Schritt (d) 5 bis 100% des in Schritt (b) von Maleinsäureanhydrid abgereicherten Absorptionsmittels abkühlt und/oder durch Verdampfung eines Teils des Absorptionsmittels aufkonzentriert.

10. Verfahren nach den Ansprüchen 1 bis 9, **dadurch gekennzeichnet, dass** man in Schritt (f) 50 bis 100% des an Fumarsäure abgereicherten Absorptionsmittels aus Schritt (d) zu Schritt (a) rückführt.

11. Verfahren nach den Ansprüchen 1 bis 10, **dadurch gekennzeichnet, dass** man als in Schritt (a) zur Absorption von Maleinsäureanhydrid aus dem Rohproduktgemisch einzusetzendes organisches Lösungsmittel ein Di-(C₁- bis C₁₂-alkyl)-phthalat einsetzt.

12. Verfahren nach den Ansprüchen 1 bis 11, **dadurch gekennzeichnet, dass** man in Schritt (c) 50 bis 100% des in Schritt (b) von Maleinsäureanhydrid abgereicherten Absorptionsmittels zu Schritt (a) rückführt.

## Claims

1. A process for decreasing fumaric acid deposits in the preparation of maleic anhydride by heterogeneously catalyzed oxidation of a hydrocarbon selected from the group of benzene, n-butane, n-butene and 1,3-butadiene with molecular oxygen in the presence of a catalyst comprising vanadium, phosphorus and oxygen, comprising
(a) the absorption of maleic anhydride from the crude product mixture in an absorbent comprising an organic solvent in an absorption column;
(b) the desorption of the maleic anhydride from the maleic anhydride-enriched absorbent obtained in step (a) in a desorption column; and
(c) the full or partial recycling of the absorbent depleted of maleic anhydride in step (b) to step (a),
wherein
(d) the entirety or a portion of the absorbent depleted of maleic anhydride in step (b), for controlled precipitation of fumaric acid, is cooled, and/or concentrated by evaporating a portion of the absorbent, to such an extent that the difference between the concentration of fumaric acid in the recycle stream at the outlet of the desorption column c(FA, desorption column outlet), under the conditions present there, in ppm by weight, and the equilibrium concentration of fumaric acid according to the solubility curve after the cooling and/or evaporation of a portion of the absorbent c(FA, equilibrium after cooling/evaporation) in ppm by weight, is greater than or equal to 250 ppm by weight and a vessel with internals is used for the precipitation and deposition of the fumaric acid, the mean residence time in the vessel with internals being from 0.05 to 6 hours;
(e) the fumaric acid precipitated as a solid as a result of the measures from step (d) is removed completely or partly, continuously or batchwise, from the absorbent recycling; and
(f) the fumaric acid-depleted absorbent from step (e) is recycled completely or partly to step (a).

2. The process according to claim 1, wherein, in step (d), the average residence time in the vessel with internals is at least 0.1 hour.

3. The process according to claims 1 and 2, wherein, in step (d), the entirety or a portion of the absorbent depleted in maleic anhydride in step (b), for controlled precipitation of fumaric acid, is cooled, and/or concentrated by evaporating a portion of the absorbent, to such an extent that the difference c(FA, desorption column outlet) minus c(FA, equilibrium after cooling/evaporation) is greater than or equal to 500 ppm by weight.

4. The process according to claims 1 to 3, wherein step (d) involves cooling to the lowest temperature within the entire absorbent recycling system.

5. The process according to claims 1 to 4, wherein step (d) involves cooling to a temperature in the range from 10 to 100°C.

6. The process according to claims 1 to 5, wherein steps (d) and (e) involve using an apparatus from which, with constant throughput of maleic anhydride-depleted absorbent, the fumaric acid deposited can be removed continuously or batchwise from the absorbent recycling system.

7. The process according to claims 1 to 6, wherein steps (d) and (e) involve using an apparatus with at least two parallel precipitation zones, from which the deposited fumaric acid, with constant throughput of the maleic anhydride-depleted absorbent through at least one of precipitation zones, can be removed batchwise from at least one other precipitation zone.

8. The process according to claims 1 to 7, wherein steps (d) and (e) involve using an apparatus with a bypass, from which the deposited fumaric acid, with constant throughput of the maleic anhydride-depleted absorbent through the bypass, can be removed batchwise from the apparatus.

9. The process according to claims 1 to 8, wherein step (d) involves cooling from 5 to 100% of the absorbent depleted in maleic anhydride in step (b) and/or concentrating it by evaporating a portion of the absorbent.

10. The process according to claims 1 to 9, wherein step (f) involves recycling from 50 to 100% of the fumaric acid-depleted absorbent from step (d) to step (a).

11. The process according to claims 1 to 10, wherein the organic solvent to be used in step (a) to absorb maleic anhydride from the crude product mixture is a di-(C₁- to C₁₂-alkyl) phthalate.

12. The process according to claims 1 to 11, wherein step (c) involves recycling from 50 to 100% of the absorbent depleted in maleic anhydride in step (b) to step (a).

## Revendications

1. Procédé pour diminuer les dépôts d'acide fumarique lors de la préparation d'anhydride de l'acide maléique par oxydation catalytique hétérogène d'un hydrocarbure, choisi dans le groupe formé par le benzène, le n-butane, le n-butène et le 1,3-butadiène, avec de l'oxygène moléculaire en présence d'un catalyseur contenant du vanadium, du phosphore et de l'oxygène, comprenant
(a) l'absorption d'anhydride de l'acide maléique à partir du mélange de produits bruts dans un absorbant contenant un solvant organique dans une colonne d'absorption ;
(b) la désorption de l'anhydride de l'acide maléique de l'absorbant enrichi en anhydride de l'acide maléique, obtenu dans l'étape (a), dans une colonne de désorption ; et
(c) le recyclage complet ou partiel de l'absorbant appauvri en anhydride de l'acide maléique dans l'étape (b) vers l'étape (a),
**caractérisé en ce qu'**on
(d) refroidit la quantité totale ou partielle de l'absorbant appauvri en anhydride de l'acide maléique dans l'étape (b), en vue de la précipitation ciblée de l'acide fumarique, et/ou concentre par évaporation une partie de l'absorbant dans une mesure telle que la différence entre la concentration en acide fumarique dans le flux recyclé à la sortie de la colonne de désorption c(AF, sortie colonne de désorption) dans les conditions qui y règnent en ppm en poids et la concentration à l'équilibre en acide fumarique selon la courbe de solubilité après le refroidissement et/ou l'évaporation d'une partie de l'absorbant c(AF, équilibre après refroidissement/évaporation) en ppm en poids est supérieure ou égale à 250 ppm en poids et on utilise, pour la précipitation et la séparation de l'acide fumarique, un récipient avec des inserts, le temps de séjour moyen dans le récipient avec des inserts étant de 0,05 à 6 heures ;
(e) élimine l'acide fumarique précipité sous forme solide par les mesures de l'étape (d), totalement ou partiellement, en continu ou de manière discontinue, du recyclage d'absorbant ; et
(f) recycle l'absorbant appauvri en acide fumarique de l'étape (e) totalement ou partiellement vers l'étape (a).

2. Procédé selon la revendication 1, **caractérisé en ce que**, dans l'étape (d), le temps de séjour moyen dans le récipient avec des inserts est d'au moins 0,1 heure.

3. Procédé selon les revendications 1 à 2, **caractérisé en ce que**, dans l'étape (d), on refroidit la quantité totale ou partielle de l'absorbant appauvri en anhydride de l'acide maléique dans l'étape (b), en vue de la précipitation ciblée de l'acide fumarique, et/ou concentre par évaporation une partie de l'absorbant dans une mesure telle que la différence c(AF, sortie colonne de désorption) - c(AF, équilibre après refroidissement/évaporation) est supérieure ou égale à 500 ppm en poids.

4. Procédé selon les revendications 1 à 3, **caractérisé en ce qu'**on refroidit, dans l'étape (d), à la température la plus basse de tout le recyclage de l'absorbant.

5. Procédé selon les revendications 1 à 4, **caractérisé en ce qu'**on refroidit, dans l'étape (d), à une température dans la plage de 10 à 100°C.

6. Procédé selon les revendications 1 à 5, **caractérisé en ce qu'**on utilise, dans l'étape (d) et l'étape (e), un appareil à partir duquel l'acide fumarique séparé peut être éliminé, en continu ou de manière discontinue, à un débit continu de l'absorbant appauvri en anhydride de l'acide maléique, du recyclage de l'absorbant.

7. Procédé selon les revendications 1 à 6, **caractérisé en ce qu'**on utilise, dans l'étape (d) et l'étape (e), un appareil présentant au moins deux zones de précipitation parallèles, à partir duquel l'acide fumarique séparé peut être éliminé, de manière discontinue, à un débit continu de l'absorbant appauvri en anhydride de l'acide maléique à travers au moins une des zones de précipitation, d'au moins une autre zone de précipitation.

8. Procédé selon les revendications 1 à 7, **caractérisé en ce qu'**on utilise, dans l'étape (d) et l'étape (e), un appareil avec une dérivation, à partir duquel l'acide fumarique séparé peut être éliminé, de manière discontinue, à un débit continu de l'absorbant appauvri en anhydride de l'acide maléique au travers de la dérivation, de l'appareil.

9. Procédé selon les revendications 1 à 8, **caractérisé en ce qu'**on refroidit, dans l'étape (d), 5 à 100% de l'absorbant appauvri en anhydride de l'acide maléique dans l'étape (b) et/ou concentre par évaporation une partie de l'absorbant.

10. Procédé selon les revendications 1 à 9, **caractérisé en ce qu'**on recycle, dans l'étape (f), 50 à 100% de l'absorbant appauvri en acide fumarique de l'étape (d) vers l'étape (a).

11. Procédé selon les revendications 1 à 10, **caractérisé en ce qu'**on utilise, comme solvant organique à utiliser dans l'étape (a) pour l'absorption de l'anhydride de l'acide maléique du mélange de produits bruts, un phtalate de di-(C₁-C₁₂-alkyle).

12. Procédé selon les revendications 1 à 11, **caractérisé en ce qu'**on recycle, dans l'étape (c), 50 à 100% de l'absorbant appauvri en anhydride de l'acide maléique dans l'étape (b) vers l'étape (a).
